# EUROPEAN PATENT APPLICATION

(11) **EP 4 494 570 A1**
(43) Date of publication of application: **22.01.2025**
(21) Application number: 24189319.7
(22) Date of filing: 18.07.2024
(51) Int. Cl.: A61B 8/08, A61B 8/12, A61B 8/00, A61B 5/00, A61N 1/00

(54) **VISUALIZATION OF DISTAL END EFFECTOR ON BIPLANE OR TRIPLANE VIEWS USING INTRACARDIAC ECHOGRAPHY (ICE)**

(30) Priority: 19.07.2023 US 202318223890
(71) Applicant: Biosense Webster (Israel) Ltd., 2066717 Yokneam (IL)
(72) Inventor: KLEMM, Ofer, 2066717 Yokneam (IL); HAIMAN, Guy, 2066717 Yokneam (IL); BREM, Erez Henri, 2066717 Yokneam (IL); BARNEA, Nadav, 2066717 Yokneam (IL); ZAR, Lior, 2066717 Yokneam (IL); ZIV-ARI, Morris, 2066717 Yokneam (IL); COHEN-SACOMSKY, Hanna, 2066717 Yokneam (IL)
(74) Representative: Carpmaels & Ransford LLP

(57) **Abstract**

A medical system includes utility and ultrasound probes, and a processor. The probes are configured for operation inside an organ. The utility probe includes a distal end effector and a first sensor configured to output first signals indicative of first positions of the distal end effector. The ultrasound probe includes ultrasound transducer array configured to image volume comprising portion of the distal end effector inside the organ, and a second sensor configured to output second signals indicative of second positions of the ultrasound transducer array. The processor is configured to, using the imaged volume, and the first and second positions, select slices of the imaged volume that comprise at least part of distal end effector in spatial relation with the organ, generate from selected slices a biplane view and/or a triplane view of the part of the distal end effector, and present the biplane view and/or triplane view to user.

## Description

### FIELD OF THE DISCLOSURE

The present disclosure relates generally to visualization of invasive medical devices, and particularly to visualization in real time of cardiac probes using an ultrasound (US) probe.

### BACKGROUND OF THE DISCLOSURE

Medical visualization using an invasive US probe was previously proposed in patent literature. For example, U.S. Patent 7,604,601 describes a medical imaging system for imaging a patient's body, including a catheter having a magnetic position sensor and an ultrasonic imaging sensor. A processor determines positional information of the portion of the catheter based on electrical signals transmitted by the position sensor. The processor displays on a display a catheter icon in the same orientation as an orientation of the portion of the catheter in the patient's body based on positional information derived from the magnetic position sensor. The processor also generates an ultrasonic image of a target based on the signals transmitted by the ultrasonic sensor and depicts in real-time the generated ultrasound image on a display in a same orientation as the orientation of the portion of the catheter in the patient's body based on positional information derived from the magnetic position sensor.

The present disclosure will be more fully understood from the following detailed description of the examples thereof, taken together with the drawings, in which:

### BRIEF DESCRIPTION OF THE DRAWINGS

Fig. 1 is a schematic, pictorial illustration of a probe-based ultrasound (US) imaging and electrophysiology (EP) mapping and ablation system, in accordance with an example of the present disclosure;
Fig. 2 is a US biplane view of arms (or splines) of the distal end effector of the EP mapping catheter of Fig. 1, as derived from two US slices selected from a volume acquired by the US probe of Fig. 1, in accordance with examples of the present disclosure; and
Fig. 3 is a flow chart that schematically illustrates a method to visualize a distal end effector in biplane or triplane US views based on selecting and image processing US slices acquired by the US probe of Fig. 1, in accordance with an example of the present disclosure.

### DETAILED DESCRIPTION OF EXAMPLES

### OVERVIEW

During a probe-based clinical procedure in a cavity of an organ of a patient, different distal end effectors of utility probes (e.g., catheters and/or other invasive probes) may be inserted into the organ.

One example of an invasive procedure is the diagnosis and treatment of arrhythmia in a cardiac chamber. In this example, an EP mapping catheter, having multiple electrodes on its distal end effector, is inserted to acquire data points used by a processor to generate an Electro-anatomical model (e.g., an EP map) of the cardiac chamber of interest. Another example(s) of such an invasive procedure is cardiac valve replacement or Left Atrial Appendage occlusion device.

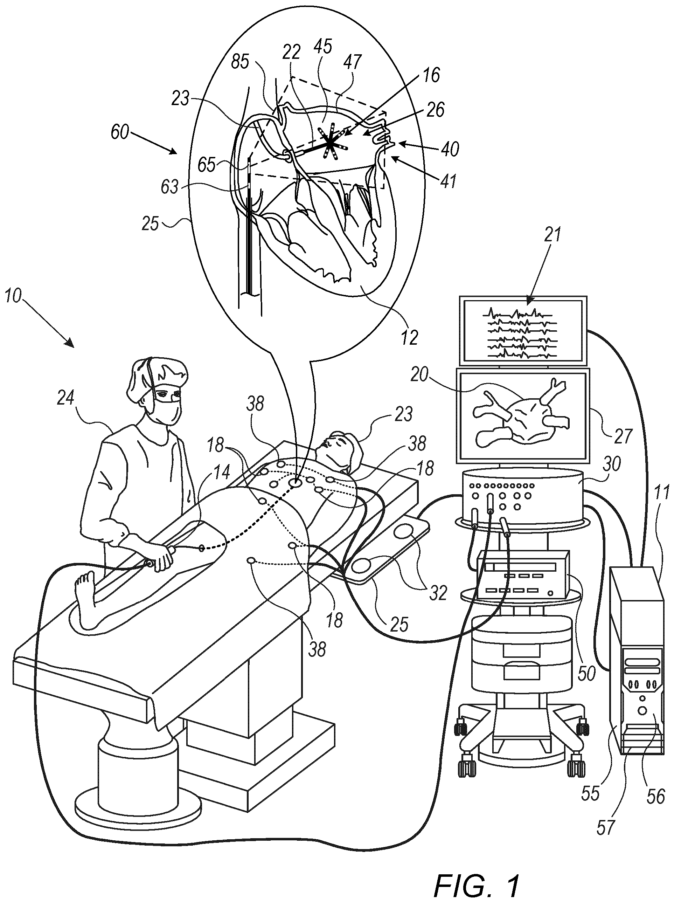

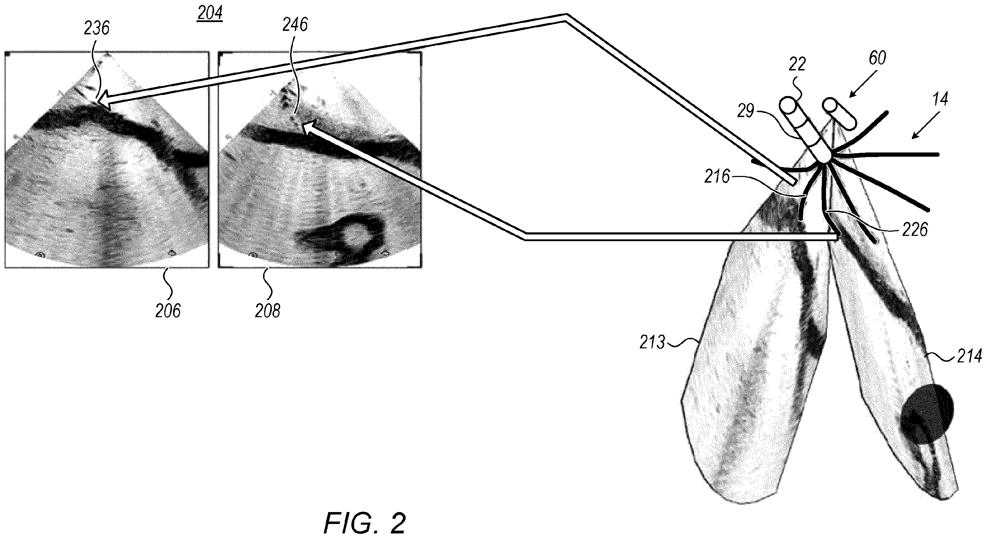

One example of a distal end effector is an expandable multi-spline assembly, such as a basket assembly. Another example is an expandable multi-arm assembly, such as the OctaRay^{®} assembly shown in Figs. 1 and 2. Yet another example is the delivery system of an artificial valve.

A utility probe, such as an EP mapping catheter or a treatment catheter such as an electrical ablation catheter, can be visualized during a clinical procedure using an ultrasound (US) probe that is also inserted into the heart.

A utility probe can also be visualized using other imaging probes such as by a trans esophageal echo (TEE) probe which may have a sensor or be location/image registered with the utility probe using another method (e.g., using anatomical landmarks)

One way of visualizing the utility probe (e.g., catheter) is to display an icon of a distal end effector of the catheter, which shows its location and orientation in relation to an anatomical model of the organ. The known dimensions and mechanics of the distal end effector, along with the monitored location of the distal end of the shaft inside the cavity, can be used to create this icon.

It was found by the inventors that it may be desirable to indicate a selected portion of the distal end effector on biplane and/or triplane views generated by the a "four-dimensional" (4D) US probe - A probe that acquires series of 3D US images over time. Specifically, providing biplane and/or triplane views can enhance a physician's ability to visualize and guide the distal end effector inside the cavity.

The biplane and triplane views are a means to visualize two imaging planes (biplane, usually but not necessarily orthogonal (can be defined by the user)) or three imaging planes (triplane, usually with 60° increments between the planes). The biplane and triplane views provide high-resolution images that can be used by the physician to monitor proper positioning and maneuvering of the distal end effector.

Examples of the present disclosure that are described herein provide a technique to visualize a distal end effector in a biplane view and/or a triplane view, based on image processing of volumetric data acquired by a 4D US probe. Typically, the technique includes selecting two or three US slices of the imaged volume, as described below.

In some examples, a medical system is provided that includes a utility probe, such as a diagnostic catheter, for insertion into a cavity of an organ. The utility probe includes (i) a distal end effector fitted at a distal end of the utility probe, and (ii) a first magnetic or electric sensor that is configured to output initial signals indicative of the first positions of the distal end effector inside the organ. The system further includes an ultrasound probe for insertion into an organ of a body, the ultrasound probe comprising (i) an ultrasound transducer two-dimensional array (Grid design) configured to image a volume of the organ, the volume comprising at least a portion of the distal end effector, and (ii) a second sensor configured to output second signals indicative of second positions of the ultrasound transducer array inside the organ. A processor of the system is configured to (i) select one or more slices of the imaged volume (using the imaged volume, the first positions, and the second positions) that comprise at least part of the distal end effector in spatial relation with the organ tissue, and (ii) generate, from the one or more selected slices, at least one of a biplane view and triplane view of that part of the distal end effector, and (iii) present at least one of a biplane view and a triplane view to a user.

The processor is configured to use the location information to select the one or more slices using an image processing algorithm, by, for example, applying thresholding to voxel values within a subset of voxels of the imaged volume voxels and identifying and calculating an embedding plane that includes the identified voxels. The resulting slice is the result of an intersection of the plane with the imaged volume at a given thickness. Registration of location data of the US probe location with that of the distal end effector location makes the selection process faster and much more efficient.

The processor is further configured to use a same or a different image processing algorithm to generate, from the one or more selected slices, at least one of the biplane view and triplane view.

In an example, the physician can indicate which arms or spline(s) of a distal end effector to display, and the system can display an icon of the specific arm or spline of interest on the slice view (biplane or triplane view) . This can provide the physician with real-time visualization of the distal end effector elements in contact with tissue, such as catheter electrodes, during navigation and confirmation of electrode-to-tissue contact, with less effort, and with less or no use of X-ray fluoroscopy. The indication for electrode-to-tissue contact is critical to successful energy delivery when treating arrhythmias.

### SYSTEM DESCRIPTION

Fig. 1 is a schematic, pictorial illustration of a probe-based ultrasound (US) imaging and electrophysiology (EP) mapping and ablation system 10, in accordance with an example of the present disclosure.

System 10 includes multiple probes (e.g., catheters), which are percutaneously inserted by physician 24 through the patient's vascular system into a chamber of interest or vascular structure of a heart 12.

The physician may initially or anytime during the procedure, insert into heart 12 a 4D US probe 60 comprising a 2D ultrasound array 65 and an integral position sensor 63. Thereafter, a location-tracked EP mapping and ablation catheter 14 is inserted via a delivery sheath 23 into a desired location in heart 12.

As seen in inset 25, the 2D ultrasound array 65 produces a 3D sector-shaped ultrasound beam 85 occupying a defined solid angle, such a beam referred to herein as a "wedge 85." With a wedge 85 beam, the 2D ultrasound array can image a substantial volume of an organ, such as an entire cardiac chamber or area sector (like the valve or the LAA) of interest (e.g., the entire left atrium 45). In Fig. 1, US probe 60 images left atrium 45 while multi-arm effector 40 is placed therein.

As further seen in inset 25, multi-arm catheter 14 includes one or more sensing electrodes 26 optionally distributed over a plurality of arms 16 at expandable distal end effector 40 and configured to sense intracardiac electrogram (IEGM) signals. Catheter 14 additionally includes a proximal position sensor (shown in Fig. 2 as a sensor 29) embedded in or near multi-arm effector 40 to track the position of a distal end of shaft 22. Optionally and preferably, this position sensor is a magnetic-based position sensor including magnetic coils for sensing three-dimensional (3D) position.

Catheter 14 itself, or another catheter to be inserted, may be used to perform ablation.

As seen in inset 25, physician 24 brings a multi-arm assembly 40 (also called hereinafter "expandable distal end assembly 40" or "distal end effector 40") fitted on a shaft 22 of catheter 14 into contact with a left atrium 45 wall 47 for sensing a target site in heart 12. For ablation, physician 24 may use the same catheter 14 or, similarly, bring a distal end of an ablation catheter to a target site. The real-time 2D ultrasound-array produced images therefore include part of the sensing electrodes 26 in contact with tissue.

Integral position sensor 63 of US probe 21 is preregistered with array 65 of the US probe. Because of the integral location sensor, the spatial coordinates of every voxel in the imaged cardiac chamber are known. Specifically, sensor 63 is configured to output first signals indicative of the location and orientation of the ultrasound transducer array 65 inside heart 12.

During the navigation of distal end 22 in heart 12, console 24 receives location and orientation signals from position sensors 63 and 29 in response to magnetic fields from external field generators 32. Magnetic field generators 32 are placed at known positions on a location pad 25 external to patient 23. These location and orientation signals are indicative of the location and orientation of ultrasound array 65 in a coordinate system of the position tracking system. Using signals from sensor 29, real-time location and orientation of the distal end of catheter 14 of shaft 22 are also calculated.

Details of the magnetic-based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

System 10 includes one or more electrode patches 38 positioned for skin contact on patient 23 to establish a location reference for location pad 25 as well as impedance-based tracking of electrodes 26. For impedance-based tracking, electrical current is directed toward electrodes 26 and sensed at electrode skin patches 38, such that the location of each electrode can be triangulated via electrode patches 38. Details of the impedance-based location tracking technology are described in US Patent Nos. 7,536,218; 7,756,576; 7,848,787; 7,869,865; and 8,456,182.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses that may be used to effect irreversible electroporation (IRE), or combinations thereof.

Patient interface unit (PIU) 30 is configured to establish electrical communication between catheters, electrophysiological equipment, power supply and a workstation 55 for controlling operation of system 10. Electrophysiological equipment of system 10 may include, for example, multiple catheters, location pad 25, body surface ECG electrodes 18, electrode patches 38, ablation energy generator 50, and recorder 11. Optionally and preferably, PIU 30 additionally includes processing capability for implementing real-time computations of catheter locations and for performing ECG calculations.

Workstation 55 includes memory 57, processor unit 56 with memory or storage with appropriate operating software loaded therein, and user interface capability. Workstation 55 may provide multiple functions, optionally including (i) modeling endocardial anatomy in three dimensions (3D) and rendering the model or anatomical map 20 for display on a display device 27, (ii) displaying on display device 27 activation sequences (or other data) compiled from recorded electrograms 21 in representative visual indicia or imagery superimposed on the rendered anatomical map 20, (iii) displaying real-time location and orientation of multiple catheters within the heart chamber, and (iv) displaying on display device 27 sites of interest such as places where ablation energy has been applied. One commercial product embodying elements of the system 10 is available as the CARTO^{™} 3 System, available from Biosense Webster, Inc., 31A Technology Drive, Irvine, CA 92618.

### VISUALIZATION OF DISTAL END EFFECTOR ON BIPLNAE/TRIPLANE US VIEWS

Fig. 2 is a US biplane view 204 of arms 236 and 246 of distal end effector 40 of the EP mapping catheter 14 of Fig. 1 as derived from two US slices 213 and 214 selected from a volume 85 acquired by the 4D US probe 60 of Fig. 1, in accordance with examples of the present disclosure. Image color is inverted for clarity.

In some examples, the physician can indicate which spline(s)/arm(s) to display (i.e., indicated arms 216 and 226), and the system can display an icon (rather than the arm itself) of the specific spline/arm of interest on the biplane view 204 or a triplane view.

As seen in Fig. 2, biplane 204 images 206 and 208 are derived (i.e., image processed) respectively from slices 213 and 214. To find and select slices 213 and 214, the processor calculates planes where the processor finds target arms (i.e., indicated arms 216 and 226) in which distal end effector 40 is embedded.

Slices 213 and 214 are then calculated as the intersection of the respective planes and image volume 85. In practice, any selected slice is taken with a given thickness (e.g., 2 mm).

Location information enables the processor to search in an efficient manner (e.g., in a sub-volume of imaged volume 85) for arms 216 and 226. To this end, the processor applies an image-processing software to the sub-volume that, for example, uses thresholding of voxel values to identify an electrode 26 in contact with wall 47 tissue. With this information the processor can then calculate the desired planes.

In Fig. 2, the location information on effector 40 is acquired using a magnetic position sensor 29 fitted on a distal end of shaft 22, and/or sensing electrodes 26 fitted on the arms of distal end effector 40. The location information on US probe 60 is acquired using magnetic position sensor 63, seen in Fig. 1.

### A METHOD FOR ESTIMATING A SHAPE OF A BASKET CATHETER PRESSED AGAINST TISSUE

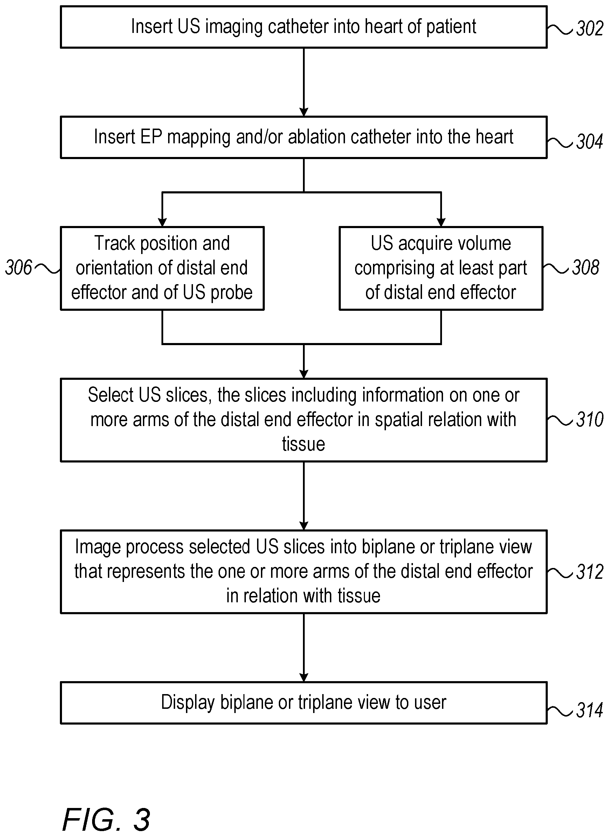

Fig. 3 is a flow chart that schematically illustrates a method to visualize a distal end effector 40 in biplane or triplane US views (e.g., biplane view 204 images 206 and 208) based on selecting and image processing US slices (e.g., slices 213 and 214) acquired by the 4D US probe 60 of Fig. 1, in accordance with an example of the present disclosure. While the method described is to a specific cardiac scenario that involves US imaging of a diagnostic and/or treatment cardiac catheter, the principles of the method hold for generating biplane or triplane views of other invasive devices caried by a utility probe.

The algorithm, according to the presented example, carries out a process that begins with physician 24 inserting intracardiac echography (ICE) 4D US probe 60 inside a cardiac chamber of heart 12, at an US probe insertion step 302.

Next, at a mapping and/or ablation catheter insertion step 304, the physician inserts a distal end effector (e.g., effector 40) into heart 12.

At a tracking step 306, using position sensors on the US probe and on the mapping and/or ablation catheter, such as magnetic sensors 29 and 63, and/or electrodes 26, processor 56 tracks the position and orientation of US probe 60 and of distal end effector 40.

In parallel, at a US acquisition step 308, processor 56 receives an acquired volume wedge 85 from 4D US probe 60 that comprises at least part of the distal end effector 40 in a spatial relation with heart tissue.

At slices selection step 310, the processor selects US slices for further image processing (based on the tracking and by running an image processing application) where the slices include information on a spatial relation between one or more arms (or splines) of the distal end effector in relation to heart tissue.

At an image processing step 312, processor 56 image processes the selected slices to generate a biplane view (e.g., view 204) or a triplane view of the one or more arms (or splines) of the distal end effector in relation to heart tissue.

Finally, at a displaying step 314, processor 56 displays the biplane or triplane view to a user (e.g., on display 27).

The flow chart shown in Fig. 3 is chosen purely for the sake of conceptual clarity. The present example may be applied, with the necessary changes made to another distal end effector, such as a basket catheter or another invasive device (e.g., a catheter delivering an artificial valve).

### EXAMPLES

### Example 1

A medical system (10) includes a utility probe (14), an ultrasound probe (60), and a processor (56). The utility probe is configured for insertion into a cavity of an organ, the utility probe comprising (a) a distal end effector (40) fitted at a distal end of the utility probe (14), and (b) a first sensor (29) configured to output first signals indicative of first positions of the distal end effector (14) inside the cavity. The ultrasound probe (60) is configured for insertion into an organ of a body, the ultrasound probe comprising (A) an ultrasound transducer array (65) configured to image a volume (85) of the organ, the volume comprising at least portion of the distal end effector (40), and (B) a second sensor (63) configured to output second signals indicative of second positions of the ultrasound transducer array (65) inside the cavity. The processor (56) is configured to, using the imaged volume (85), the first positions, and the second positions, (i) select one or more slices (213, 214) of the imaged volume (85) that comprise at least part of the distal end effector (40) in spatial relation with the organ, (ii) generate from the one or more selected slices at least one of a biplane view (204) and triplane view of the part of the distal end effector, and (iii) present the at least one of the biplane view (204) and triplane view to user.

### Example 2

The system (10) according to example 1, wherein the part of the distal end effector (40) comprises one of expandable splines and expandable arms (16).

### Example 3

The system (10) according to example 1, wherein the part of the distal end effector comprises an artificial valve.

### Example 4

The system (10) according to any of examples 1 through 3, wherein the ultrasound probe (60) is an intracardiac echography (ICE) 4D ultrasound (US) catheter.

### Example 5

The system (10) according to any of examples 1 through 4, wherein the first and second sensors (29, 63) are magnetic position sensors configured to generate the first and second signals in response to a magnetic field applied by a position tracking system.

### Example 6

The system according to any of examples 1 through 4, wherein the first sensor is an electrode (26) of the distal end effector configured to generate the first signals as part of an electrical position tracking system.

### Example 7

The system (10) according to any of examples 1 through 6, wherein the processor is configured to select one or more slices (213, 214) using an image processing algorithm.

### Example 8

The system (10) according to any of examples 1 through 7, wherein the processor is configured to generate from the one or more selected slices (213, 214) at least one of a biplane view (204) and triplane view using an image processing algorithm.

### Example 9

The system (10) according to any of examples 1 through 8, wherein the cavity of the organ is a cardiac chamber of a heart (12).

### Example 10

A medical method includes inserting a utility probe (14) into a cavity of an organ, the utility probe comprising (a) a distal end effector (40) fitted at a distal end of the utility probe (14), and a first sensor (29) configured to output first signals indicative of first positions of the distal end effector (40) inside the cavity. An ultrasound probe (60 is inserted into an organ of a body, the ultrasound probe (60) comprising an ultrasound transducer array (65) configured to image a volume (85) of the organ, the volume comprising at least portion of the distal end effector (40), and a second sensor (63) configured to output second signals indicative of second positions of the ultrasound transducer array (65) inside the cavity. Using the imaged volume, the first positions, and the second positions, one or more slices (213, 214) of the imaged volume (85) are selected, that comprise at least part of the distal end effector (40) in spatial relation with the organ. At least one of a biplane view (204) and triplane view of the part of the distal end effector (40) are generated from the one or more selected slices (213, 214). The at least one of the biplane view (204) and triplane view are presented to a user.

### Example 11

The method according to example 10, wherein the part of the distal end effector comprises one of expandable splines and expandable arms.

### Example 12

The method according to example 10, wherein the part of the distal end effector comprises an artificial valve.

### Example 13

The method according to example 10, wherein the ultrasound probe is an intracardiac echography (ICE) 4D ultrasound (US) catheter.

### Example 14

The method according to example 10, wherein the first and second sensors are magnetic position sensors configured to generate the first and second signals in response to a magnetic field applied by a position tracking system.

### Example 15

The method according to example 10, wherein the first sensor is an electrode of the distal end effector configured to generate the first signals as part of an electrical position tracking system.

### Example 16

The method according to example 10, wherein selecting one or more slices comprises using an image processing algorithm.

### Example 17

The method according to example 10, wherein generating from the one or more selected slices at least one of a biplane view and triplane view comprises using an image processing algorithm.

### Example 18

The method according to example 10, wherein the cavity of the organ is a cardiac chamber of a heart.

Although the examples described herein mainly address cardiac diagnostic applications, the methods and systems described herein can also be used in other medical applications.

It will be appreciated that the examples described above are cited by way of example, and that the present disclosure is not limited to what has been particularly shown and described hereinabove. Rather, the scope of the present disclosure includes both combinations and sub combinations of the various features described hereinabove, as well as variations and modifications thereof which would occur to persons skilled in the art upon reading the foregoing description and which are not disclosed in the prior art.

## Claims

1. A medical (10) system, comprising:
a utility probe (14) for insertion into a cavity of an organ, the utility probe comprising:
a distal end effector (40) fitted at a distal end of the utility probe (14); and
a first sensor (29) configured to output first signals indicative of first positions of the distal end effector (14) inside the cavity;
an ultrasound probe (60) for insertion into an organ of a body, the ultrasound probe (60) comprising:
an ultrasound transducer array (65) configured to image a volume (85) of the organ, the volume comprising at least portion of the distal end effector (40); and
a second sensor (63) configured to output second signals indicative of second positions of the ultrasound transducer array (65) inside the cavity; and
a processor (56), which is configured to:
using the imaged volume (85), the first positions, and the second positions, select one or more slices (213, 214) of the imaged volume (85) that comprise at least part of the distal end effector (40) in spatial relation with the organ;
generate from the one or more selected slices at least one of a biplane view (204) and triplane view of the part of the distal end effector; and
present the at least one of the biplane view (204) and triplane view to a user.

2. The system (10) according to claim 1, wherein the part of the distal end effector (40) comprises one of expandable splines and expandable arms (16).

3. The system (10) according to claim 1, wherein the part of the distal end effector comprises an artificial valve.

4. The system (10) according to any preceding claim, wherein the ultrasound probe (60) is an intracardiac echography (ICE) 4D ultrasound (US) catheter.

5. The system (10) according to any preceding claim, wherein the first and second sensors (29, 63) are magnetic position sensors configured to generate the first and second signals in response to a magnetic field applied by a position tracking system.

6. The system according to any of claims 1 to 4, wherein the first sensor is an electrode (26) of the distal end effector configured to generate the first signals as part of an electrical position tracking system.

7. The system (10) according to any preceding claim, wherein the processor is configured to select one or more slices (213, 214) using an image processing algorithm.

8. The system (10) according to any preceding claim, wherein the processor is configured to generate from the one or more selected slices (213, 214) at least one of a biplane view (204) and triplane view using an image processing algorithm.

9. The system (10) according to any preceding claim, wherein the cavity of the organ is a cardiac chamber of a heart (12).
